# EUROPEAN PATENT APPLICATION

(11) **EP 4 782 014 A1**
(43) Date of publication of application: **29.07.2026**
(21) Application number: 25153614.0
(22) Date of filing: 23.01.2025
(51) Int. Cl.: A61L 2/04, A47L 15/00, A47L 15/42, A47L 15/50

(54) **ACCESSORY FOR APPARATUS FOR WASHING AND DISINFECTING MEDICAL ARTICLES**

(71) Applicant: Bonferraro S.p.A., 37060 Bonferraro di Sorgà (VR) (IT)
(72) Inventor: GOBBI, Ezio, Roncoferraro MN (IT)
(74) Representative: Concone, Emanuele

(57) **Abstract**

An article support for an apparatus for washing and disinfecting medical articles includes a base (1) with a central vertical hole (1A) and a plurality of flexible arms (2) extending upward from the base (1), the support having a modular structure in which the arms (2) are not integral with the base (1) but reversibly connected thereto through coupling means (1B, 1C, 2A, 2B, 3).

## Description

The present invention relates to apparatuses for washing and disinfecting (thermodisinfectors) medical articles such as laboratory glassware, robotic surgical instruments, laparoscopic cannulas, and the like, and in particular to an accessory to be mounted on such apparatus to support the articles during the operating cycle. In the following, specific reference will be made to glassware, but it is clear that the above can be applied to supports for any type of medical article.

It is well known that an apparatus for washing and thermodisinfecting medical articles of this type traditionally includes at least one trolley with a washing manifold, equipped with a plurality of branches each with a plurality of attachments for mounting elongated vertical nozzles, as well as an overlying frame for supporting glassware. Specifically, glassware (e.g., bottles or flasks) are inserted upside down onto the nozzles, and the frame serves to prevent them from falling against the nozzle and/or other adjacent glassware during the washing cycle. An example of such a trolley is the LB16I model from the company Smeg S.p.A. (Guastalla, Italy).

This simple configuration is robust and inexpensive but has significant limitations in functionality, since the rigid square-mesh structure of the frame has no margin of adaptability to glassware of different shapes and/or sizes. As a result, it is optimal only for specific models of glassworks, while for other models the frame meshes may be too large or too small, or placed at an inappropriate height.

A solution with greater functionality is to use, instead of the frame, individual glassware supports that are attached to each nozzle. These supports can then be different depending on the type of glassware and/or its size, and can be easily replaced if the support breaks or if the type of glassware to be washed changes, thus being able to wash different glassware on the same trolley.

In practice, this type of support consists of a base from which extend upward a plurality of flexible support arms, typically three or four, which deform outward to accommodate and enclose the glassware. The base has a hole for mounting on the nozzle and a fastening system, such as a screw or spring clip, to secure the support on the nozzle at the desired height. In this way, the glassware is inserted onto the nozzle until its mouth abuts on the base of the support, and the flexible arms of the support are pushed outward by the glassware itself so as to fit its shape and size.

The support can be made of plastic-coated stainless steel wire, like a common dishwasher basket, or it can be a monolithic piece molded from plastic. Examples of such types of supports are, respectively, accessories No. 4424800 and No. 4585204 shown in the brochure *"FlaskScrubber*^{®} & *SteamScrubber*^{®} *Laboratory Glassware Washers"* by the company LABCONCO CORPORATION (Kansas City, MO - USA).

This known solution is a significant improvement over the aforementioned simple square-mesh frame, but it still has several drawbacks due to the monolithic structure of the support and the way it is mounted on the trolley.

In the first place, the monolithic structure of the support makes its complete replacement unavoidable if even one of its components, typically one of the support arms, is damaged or broken. In addition, the mold for the production of a plastic support is very expensive due to the complex shape of the support, so making a full range of supports of various shapes and sizes requires a significant economic investment.

Secondly, the plastic material used for the support must be a compromise between the conflicting requirements of the different parts of the support, since the base must be more rigid and sturdy for mounting on the nozzle while the arms must be more flexible for simple and effective accommodation of the glassware. As a result, the material used is necessarily not optimal for either function, with the risk that the base may be too weak to withstand repeated stresses due to deformation of the arms and high-temperature thermal cycling for thermodisinfection of the glassware, or the arms may be too rigid for easy insertion of the glassware with the risk of damage to the glassware and/or breakage of the arms themselves due to excessive deformation.

The presence of the fastening system for mounting the support on the nozzle also contributes to the complexity, and thus higher cost, of prior art supports. Moreover, the support is constrained to the diameter of the nozzle on which it is to be mounted, since the base must have a hole of corresponding diameter, so it cannot be used either on larger diameter nozzles on which it cannot fit, or on smaller diameter nozzles on which the clearance between base and nozzle would create an unstable connection.

It is therefore the purpose of the present invention to provide a support for glassware that is free from such drawbacks. Said purpose is achieved by means of a modular support comprising a plurality of flexible arms and a base which are provided with means for reversible mutual coupling, the base being preferably provided with a hole suitable for direct mounting on the wash manifold attachment of the trolley. Other advantageous features of the present support are specified in the dependent claims.

The main advantage of the present support is that of being modular, so that it is cheaper to produce and more functional in use since the molds for the base and arms are much simpler and the individual components of the support can be replaced in case of breakage or change in the type of glassware to be washed. It is also possible to remove one or more arms of the support to adapt it to particular shapes or sizes of glassware without having to use another specific support. This also results in greater flexibility in the design of the support, allowing for a wider range of supports, both in shape and size, without requiring an excessive economic investment.

This also brings a second significant advantage of being able to use optimal materials for the different functions performed by the different parts of the support, so that the base can be molded using a more rigid and robust plastic and the arms using a more flexible plastic. It is also possible to use different materials, such as steel for the base and plastic for the arms, or vice versa, depending on specific design requirements.

The preferred embodiment in which the base is configured for direct mounting on the wash manifold attachment of the trolley has additional advantages, starting with the elimination of the attachment system on the nozzle, which further simplifies the base and makes it cheaper to manufacture. In fact, in this embodiment the support is attached to the trolley directly with the bushing usually used to mount the nozzle on the wash manifold, so that this bushing assumes the dual function of a fastening system for both the nozzle and the support, and the latter can dispense with its own fastening system.

A further notable advantage of this embodiment comes from the fact that the support is no longer constrained to the diameter of the nozzle, so that the same support can be used in combination with nozzles of different diameters.

Further advantages and features of the support according to the present invention will be apparent to those skilled in the art from the following detailed description of an embodiment thereof with reference to the attached drawings in which:

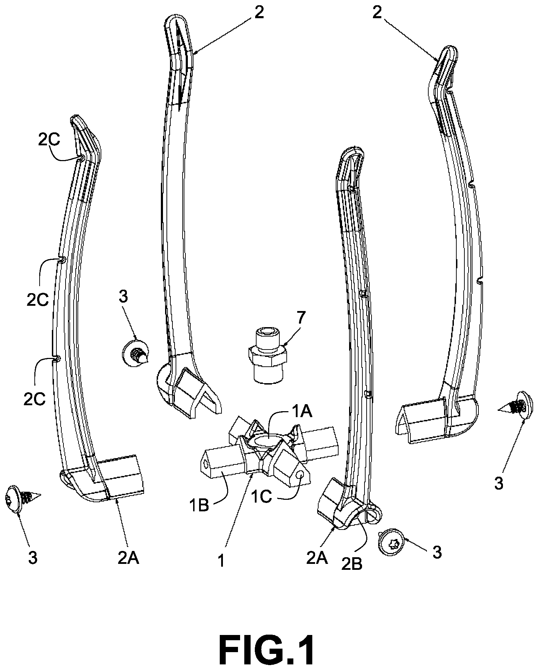

Fig.1 is an exploded perspective view of a support and the bushing used to mount it on the trolley;
Fig.2 is a perspective view of the support of Fig. 1 in assembled condition and mounted on the trolley;
Figs.3-5 are perspective views of the support of Fig. 1 in assembled condition combined with different types of nozzles that differ in the type of abutment for the glassware mouth; and
Figs.6-9 show the support supporting different types of glassware.

Referring to the above figures, it can be seen that a support according to the invention traditionally comprises a base 1 and a plurality of flexible arms 2 extending upward, four in the example shown, the base 1 having a central vertical hole 1A.

A first innovative aspect of the present support, as mentioned above, lies in its modular structure, as the arms 2 are not integral with the base 1 as in prior art supports, but reversibly connected to it through coupling means. More specifically, in the illustrated embodiment, base 1 has as coupling means horizontal connectors 1B equipped with an axial hole 1C, while arms 2 have corresponding horizontal feet 2A that fit over connectors 1B and are equipped with corresponding axial holes 2B for attachment by means of screws 3 that engage holes 1C.

It follows that base 1 can receive arms 2 of different shapes, sizes, and materials as long as they are equipped with the corresponding feet 2A, thus allowing for a wide range of supports adaptable to a wide variety of glassware shapes and sizes by combining parts that are simple and inexpensive to produce and that can be easily substituted from time to time as needed.

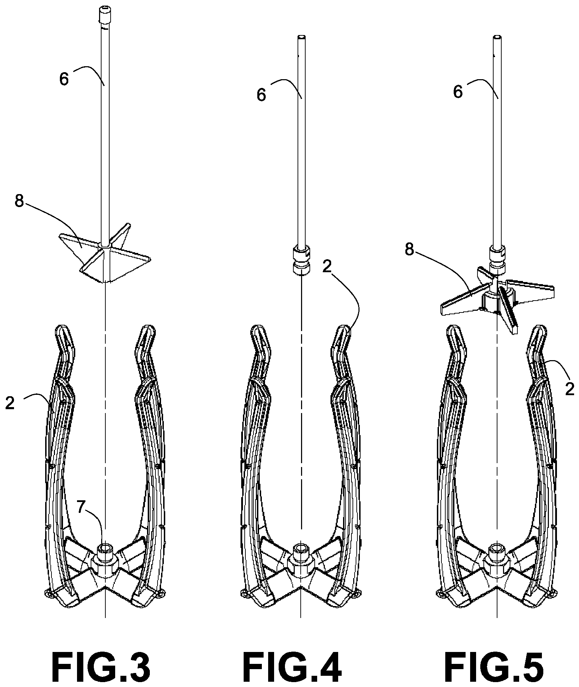

Another preferable innovative aspect of the present support is to have hole 1A sized for mounting directly on attachment 4 of manifold 5 of the wash trolley, instead of on nozzle 6. Thus, the mounting of the support is achieved by means of bushing 7 that is usually used only for mounting nozzle 6 on attachment 4. In this way, as mentioned earlier, the support is no longer constrained to the diameter of nozzle 6, so it can be used in combination with different types of nozzles 6 that may have different diameters and/or other different characteristics, such as the type of abutment 8 for the glassware mouth illustrated in Figures 3 to 5.

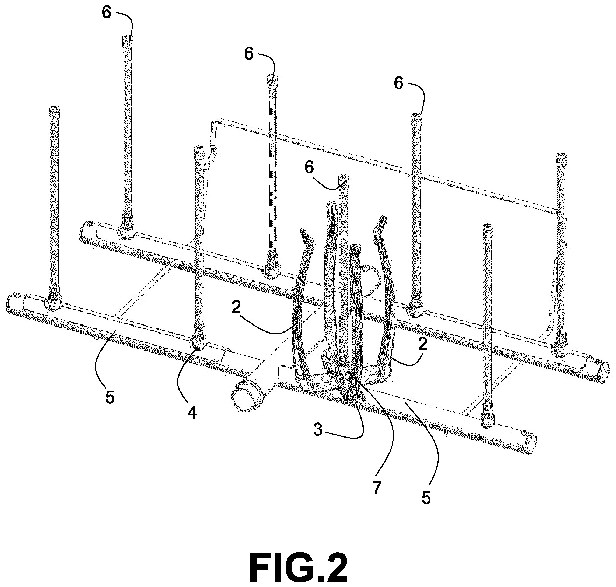

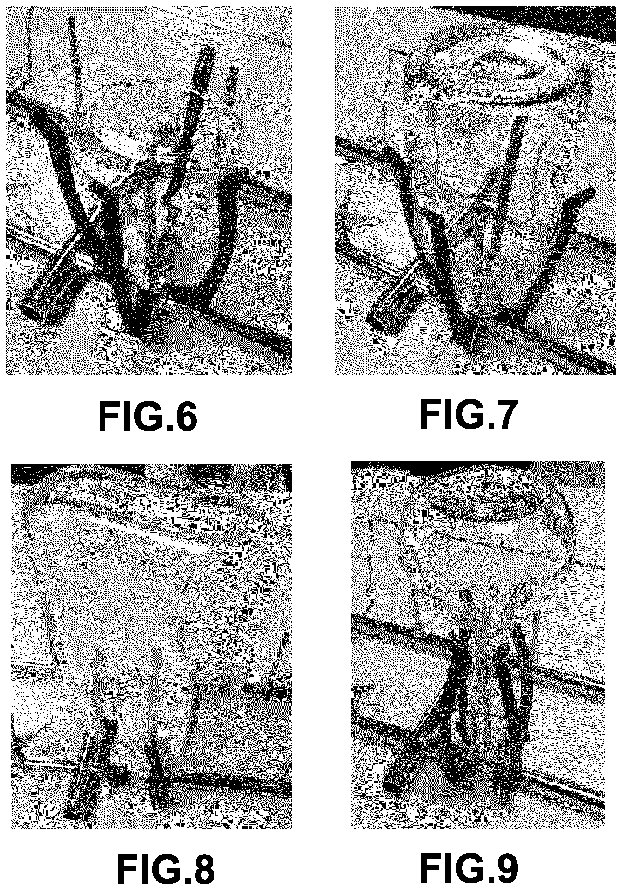

As best illustrated in Figures 6 to 8, the arms 2 are spread apart to a greater or lesser extent depending on the type of glassware accommodated by the support. However, in the case of thin glassware with a narrow and long mouthpiece smaller than the distance between the arms in the rest position in figures 2 to 5, it may be useful to flex the arms 2 inward to provide more stability to the glassware during washing, as shown in Fig.9.

For this purpose, arms 2 are preferably provided with one or more notches 2C arranged at different heights, the same heights on all arms 2, in which an elastic ring or elastic C-clip (schematically represented by the red lines) can be engaged so as to make arms 2 converge on the thin glassware.

It is clear that the above-described and illustrated embodiment of the support according to the invention is just an example susceptible of various modifications. In particular, the means for reversible coupling between base 1 and arms 2 can be realized in many other ways known to one skilled in the art, such as interlocking assembly, bayonet assembly, direct screwing of arms 2 onto base 1, etc.

Similarly, the number and/or shape of arms 2 and the corresponding connectors on base 1 can be freely varied according to specific operational requirements, and also notches 2C can be replaced by equivalent engagement elements for the elastic element, e.g., raised C-shaped seats.

## Claims

1. Article support for apparatus for washing and disinfecting medical articles, said support comprising a base (1) with a central vertical hole (1A) and a plurality of flexible arms (2) extending upward from said base (1), **characterized in that** the support has a modular structure in which said arms (2) are not integral with the base (1) but reversibly connected thereto through coupling means.

2. Support according to claim 1, **characterized in that** the coupling means of the base (1) are horizontal connectors (1B) provided with an axial hole (1C), and the coupling means of the arms (2) are corresponding horizontal feet (2A) that fit on said horizontal connectors (1B) of the base (1) and are provided with corresponding axial holes (2B) for attachment by screws (3) engaging said axial holes (1C) of the horizontal connectors (1B) of the base (1).

3. Support according to claim 1 or 2, **characterized in that** the base (1) is made of a material different from the material of the arms (2).

4. Support according to any of the preceding claims, **characterized in that** the arms (2) are provided with one or more seats, preferably notches (2C), arranged at different heights, the same heights on all the arms (2), and the support also comprises at least one elastic element suitable for engaging said seats so as to make the arms (2) converge with respect to their rest position.

5. Trolley for apparatus for washing and disinfecting medical articles, said trolley comprising a washing manifold (5) having a plurality of branches each having a plurality of attachments (4) for mounting elongated vertical nozzles (6) by means of bushings (7), as well as supports for supporting said medical articles, **characterized in that** said supports are supports according to any of the preceding claims, **and in that** the bases (1) of the supports have the central vertical hole (1A) sized to fit on said attachments (4) and to be fixed on the trolley by means of said bushings (7).
